# EUROPEAN PATENT SPECIFICATION

(11) **EP 3 102 271 B1**
(45) Date of publication and mention of the grant of the patent: **30.09.2020**
(21) Application number: 15720727.5
(22) Date of filing: 07.05.2015
(51) Int. Cl.: A61M 16/20, A61M 16/08

(54) **DEVICE AND METHOD FOR A LOW RESISTANCE VALVE**
VORRICHTUNG UND VERFAHREN FÜR EIN VENTIL MIT GERINGEM WIDERSTAND
DISPOSITIF ET PROCÉDÉ POUR UNE VALVE À FAIBLE RÉSISTANCE

(30) Priority: 07.05.2014 EP 14167428
(43) Date of publication of application: 14.12.2016
(73) Proprietor: Shenzhen Mindray Bio-Medical Electronics Co., Ltd., Shenzhen 518057 (CN)
(72) Inventor: LAKSOV, Joakim, 182 56 Danderyd (SE); RINGDEN, Ola, 172 37 Sundbyberg (SE)
(74) Representative: KIPA AB
(86) International application number: PCT/EP2015/060005
(87) International publication number: WO 2015/169881

(56) References cited:
- EP-A1- 1 897 578
- EP-A1- 2 085 106
- EP-A1- 2 236 881
- WO-A1-99/59517
- WO-A1-02/051486
- WO-A1-2009/089807
- US-A- 3 672 366
- US-A- 4 699 137
- US-A- 5 127 400
- US-A1- 2001 017 135
- US-A1- 2002 121 275
- US-A1- 2007 074 724
- US-A1- 2008 264 412
- US-A1- 2011 126 836
- US-A1- 2011 239 733
- US-A1- 2013 269 686
- US-B1- 6 446 629
- US-B2- 7 066 177

## Description

### Field of the Invention

The present invention relates to a valve for a respiratory device, one example of such valves are expiration and inspiration valves. Respiratory devices are, for example, anaesthetic machines, medical ventilators etc. More particularly, the invention relates to a valve device for controlling the flow of at least one fluid through at least one channel. In particular it relates to reduce the flow resistance and the pressure drop through a valve, such as expiration valves or inspiration valves.

### Description of the Prior Art

It is known that when designing low pressure valves, especially in the field of gas control in medical ventilators, is it of high importance that the flow channel in the valve has low flow resistance, low pressure drop and no or little turbulence. Also, it is often desirable for the design of the valve to be small and light.

The most common design of low pressure expiration valves comprises a circular disk lying against the end of a tube forming a valve seat. US patent 5,127,400 discloses an example of such a design. These valves are generally devised as disk valves, i.e. the directional valve has a disk-shaped valve body that rests loosely on a valve seat.

US 4,699,137 discloses an exhalation valve is disclosed having an inlet, an outlet and a diaphragm movable between closed position covering the inlet and an open position away from the inlet.

US 6,446,629 B1 discloses a valve made of two pipes and that has a flow rate control electromagnetic valve which is controlled by a pressure sensor for synchronization with oscillating air pressure.

US 3,672,366 discloses an apparatus for producing intermittent positive pressure comprises a high-pressure nutrient gas supply. The apparatus may include a valve device for controlling a gas flow through a conduit and opens and closes with relatively sudden pressure change. The valve device can include a housing having a tubular, high-pressure gas inlet and a low-pressure gas outlet, and a member in the housing, co-axial with the inlet, for opening and closing the valve.WO 99/59517 discloses an exhalation valve assembly in which a PEEP valve and an exhalation valve are combined into a single valve mechanism. The assembly includes a wye in which a patient tube splits at equal angles into the ventilator tube and a tube closed off by the PEEP valve.

An improved valve with reduced flow resistance and lower pressure drop would be advantageous. It would be further advantageous if the valve had an improved stability thereby reducing the likelihood of the valve starting to oscillate when connecting means with a pressure drop after the valve.

### SUMMARY OF THE INVENTION

Accordingly, examples of the present disclosure preferably seek to mitigate, alleviate or eliminate one or more deficiencies, disadvantages or issues in the art, such as the above-identified, singly or in any combination by providing a device, or method according to the appended patent claims for providing an improved valve for a respiratory device, such as an expiration valve or inspiration valve. The valve may be a disk-valve but may equally be other types of valves. Disclosed herein are device and methods for providing the improved valve.

According to the invention, there is provided a valve according to claim 1, a kit according to claim 10, and a method according to claim 11. The dependent claims 2-9 and 12 define preferred embodiments of the invention.

It should be emphasized that the term "comprises/comprising" when used in this specification is taken to specify the presence of stated features, integers, steps or components but does not preclude the presence or addition of one or more other features, integers, steps, components or groups thereof.

### BRIEF DESCRIPTION OF THE DRAWINGS

These and other aspects, features and advantages of which examples of the disclosure are capable of will be apparent and elucidated from the following description of embodiments of the invention and of comparative examples, reference being made to the accompanying drawings, in which
Figs. 1A to 1D are illustrating schematic overviews of an exemplary disk-valve according to an embodiment of the invention;
Figs. 2A to 2D are illustrating schematic overviews of an exemplary disk-type valve according to an embodiment of the invention;
Figs. 3A and 3B are illustrating a schematic example of relation between areas of a round and a rectangular check-valve and the length of the valve housing in a valve according to an embodiment of the invention;
Fig. 4 is illustrating a schematic overview of an exemplary disk-type valve not according to the invention;
Figs. 5A and 5B are illustrating two exemplary valves of the disclosure, Fig. 5A with a circular outlet (not according to the invention) and Fig. 5B with a rectangular outlet (according to an embodiment of the invention);
Figs. 6A and 6B are illustrating two exemplary valves of the disclosure, Fig. 6A with a circular outlet (not according to the invention) and Fig. 6B with a rectangular outlet (according to an embodiment of the invention).

### DESCRIPTION OF EXAMPLES

Specific examples of the disclosure will now be described with reference to the accompanying drawings. These examples are provided so that this disclosure will be thorough and complete, and will fully convey the scope of the disclosure to those skilled in the art.

The following description focuses on an example of the present disclosure applicable to a valve for a respiratory device. For example, the valve may be used in devices for patient monitoring, anaesthesia, medical ventilators, etc. However, it will be appreciated that the description is not limited to this application but may be applied to many other systems where a fluid pump is required.

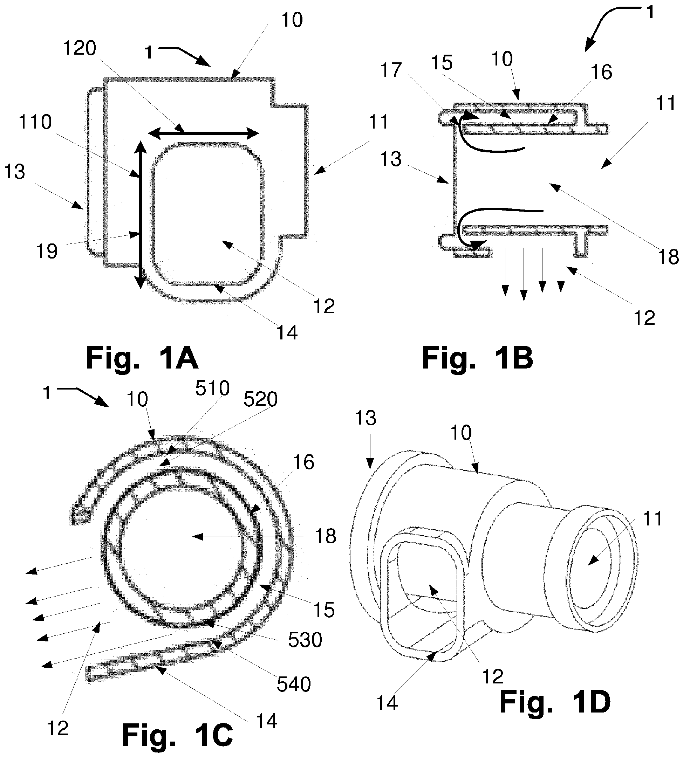

Figure 1A illustrates an exemplary side view of a valve 1 according to an embodiment of the invention. The valve has a valve housing 10 with an outlet 12 and an inlet 11. The outlet as illustrated in the figures 1A to 1D, has at least one portion 14 that goes all the way out to a chamber edge or close to the chamber edge. The height of the outlet is in the illustrated example, at least half the height of the chamber. In the illustration the outlet 12 is illustrated as rectangular, but other non-circular regular shapes may be possible such as, for example, the outlet 12 being elliptical (in a valve not according to the invention), oblong, square, or a polygon.

Figure 1B illustrates an exemplary cross sectional view of the valve 1 in figure 1A. The valve 1 has a valve housing 10. The valve housing 10 has an inner tubular and cylindrical channel. A tubular flow channel 18 is arranged inside the valve housing so that a space between an inner surface of the valve housing 10 and an outer surface 16 of the tubular flow channel 18 forms a chamber 15.

An inlet 11 is, when in use, in a fluid connection via a conduit or a tube to a patient connected to a medical ventilator or anaesthesia machine. The valve 1 may be used either as an expiration valve or inspiration valve.

The inlet 11 forms an inlet to the valve 1.

An outlet 12 is arranged at the side of the valve housing 10, perpendicular to the inlet 11.

At an opposite side to the inlet 11 on the tubular flow channel 18, the edges are configured to be a valve seat 17. A valve body 13 is arranged to be resting at the valve seat 17 when the valve is closed. When the valve is open there is a gap between the valve body 13 and the valve seat 17 allowing fluid to flow from the tubular flow channel 18 into the chamber 15. The fluid thereafter flows from the chamber out through the outlet 12. In this example is the valve body 13 illustrated as disk-shaped, but other designs are possible as long as it is configured to open and close the valve. The surface of the valve body 13 which may be in contact with the valve seat 17 should be made of a soft material.

Some improvements over a standard disk-valve are achieved by arranging at least one portion 14 of the outlet housing to go all the way out to a chamber edge or close to a chamber edge. This may yield a lower pressure drop and lower flow resistance than for a standard arrangement of an outlet for a disk- type valve. Also, by having a non-circular regular shaped outlet, such as, for example, a rectangular, elliptic (in a valve not according to the invention), oblong, square, or a polygon, the cross-sectional flow area of the chamber 15 may be reduced in size compared to a normal disk-valve with a circular outlet as the outlet having a non-circular regular shape may be designed with a larger area increasing the flow out from the chamber. A smaller cross-sectional flow area of the chamber 15 increases the stability of the valve. Problems with instability may lead to oscillation of the valve if means with a pressure drop is connected to the outlet.

Stability is particularly important if such means with a pressure drop, for example be flow measuring means, a check-valve or an evacuation apparatus or tube for gases, are connected to the valve.

An advantage with achieving a larger cross-sectional flow area of the outlet is that a check-valve connected to the outlet will have the same larger area. Hence pressure drop of the check-valve may also be reduced.

Figure 1C is illustrating a schematic cross-sectional view of the valve 1. As illustrated in this example of a disk-valve, a valve housing 10 has an inner tubular or cylindrical channel. A tubular flow channel 18 is arranged inside the valve housing 10 so that a space between an inner surface of the valve housing 10 and an outer surface 16 of the tubular flow channel 18 forms a chamber 15. The valve housing 10 has an outlet 12. The outlet has at least one portion 14 that goes all the way out to a chamber edge or close to the chamber edge.

By arranging the at least one portion 14 of the outlet to go all the way out to a chamber edge or close to the chamber edge, the portion 14 may be arranged so that at least a portion of an inner surface of the outlet housing is connected with the inner surface of the valve housing at a first transition portion. In some examples this means that the first transition portion may be arranged, in relation to the chamber 15, between a first extreme point 520 of the outer surface of the tubular channel and a first point 510 of the inner surface of the valve housing positioned at an opposite side of the chamber 15.

Depending on the direction the valve is seen, this may also be described as the first transition portion being arranged, in relation to the chamber 15, between a maximum point 520 of the outer surface of the tubular channel and a maximum point 510 of the inner surface of the valve housing. Alternatively and/or additionally, depending on the direction of the valve is seen, the first transition portion may be arranged, in relation to the chamber 15, between a minimum point 530 of the outer surface of the tubular channel and a minimum point 540 of the inner surface of the valve housing.

Additionally and/or alternatively, in some examples, when arranging the at least one portion 14 of the outlet to go all the way out to a chamber edge or close to the chamber edge, the transition portion may be located inside the chamber 15 so that a portion of an inner surface of the outlet housing may extend out from the chamber 15. In this way at least a portion of the inner surface of the outlet housing extends into the chamber 15 forming a part of the inner surface of the valve housing.

By arranging the at least one portion 14 of the outlet housing to go all the way out to a chamber edge or close to the chamber edge, the at least one portion of the inner surface of the outlet housing is connected to the inner surface of the valve housing so that the at least one portion 14 of the inner surface of the outlet housing is arranged tangential to the chamber 15, and tangential to the inner surface of the valve housing as illustrated in the example in figure 1C.

Figure 1D is illustrating a three-dimensional view over the exemplary disk-type valve illustrated in figures 1A to 1C.

Arranging the at least one portion 14 of an inner surface of the outlet housing, as described herein in relation to Figure 1A to 1D, allows a fluid to flow out from the chamber 15 in a tangent direction along the inner surface of the outlet housing.

To further improve the flow out from the chamber 15, and thereby reduce the flow resistance and the pressure drop of the valve 1, one portion of the outlet 12 is planar and parallel to an axial direction of the tubular flow channel and valve housing. The reduced flow resistance and pressure drop is further enhanced as the planar portion of the outlet 12 is the same as the portion 14 which is connected to the inner surface of the valve housing 10.

The planar side which is parallel to the axial direction of tubular flow channel 18, or valve housing, may create a straight flow path out of the chamber 15. Additionally, a wider 120 planar side of the outlet 12 creates a larger cross-sectional flow area along the planar side in direct fluid communication with the chamber 15 compared to a circular outlet. The outlet may be as wide as the length of the valve housing 10. A planar side, according to this arrangement, allows the flow out from the chamber 15 to increase and have a more homogenous velocity profile of the flow compared to a circular outlet. Additionally, due to the improved flow out from the chamber 15, the cross-sectional flow area of the chamber 15 may be made smaller than for a conventional disk-valve with a circular outlet. A smaller cross-sectional flow area of the chamber 15 may improve the stability of the valve 1. Hence the valve may be less likely to start to oscillate.

Additionally, in some example of the valve 1, to improve the flow out from the chamber 15 and thereby reduce the flow resistance and the pressure drop of the valve 1, one portion of the outlet 12 may be planar or straight, and parallel to the valve seat 17, such as parallel to the radial direction of the flow channel, or the valve housing.

The planar side 19, which is parallel to the valve seat 17 (such as parallel to the radial direction of the flow channel, or the valve housing), may create a straight flow path out of the chamber 15 along the planar or straight side. Additionally, a longer height 110 of the planar or straight portion 19 may further improve the fluid flow out of the chamber 15, hence reduce the resistance. The highest improvement is archived if the planar or straight side portion 19 of the outlet 12, parallel to the valve seat 17, is the side of the outlet closest to the valve seat 17.

Additionally, a large outlet area lowers the pressure drop. When having a non-circular regular shaped outlet area, such as, for example, a rectangular, oblong, elliptically (in a valve not according to the invention) or polygonal shaped, a large area of the outlet may effectively be created compared to a circular outlet. Thus the flow out from the chamber 15 may increase and have a more homogeneous velocity profile of the flow compared to a circular outlet.

Additionally, due to the improved flow out from the chamber 15, the cross-sectional flow area of the chamber 15 may be made smaller than for a conventional disk-valve with a circular outlet. A smaller cross-sectional flow area of the chamber 15 may improve the stability of the valve 1. Hence the valve may be less likely to start to oscillate.

Additionally, in some examples of the disclosure, the outlet 12 may be rectangular. A rectangular outlet 12, as illustrated in the figures, may further enhance the flow out from the chamber 15 compared to only having one side that is planar or straight as the cross-sectional flow area may be made even larger. Hence the pressure drop and the flow resistance may be further reduced. A rectangular outlet 12 may also have an even more homogenous velocity profile of the flow compared to if only one side is planar or straight. Also due to the improved flow out from the chamber 15 with a rectangular outlet 12, the cross-sectional flow area of the chamber 15 may be even further reduced compared to when only one side is planar or straight.

Additionally, by using a rectangular shaped outlet 12, the outlet may be optimized so that the sides of the rectangular outlet is as far away as possible from the center of the outlet 12, which may further improve the homogeneity of the fluid flow out from the outlet 12.

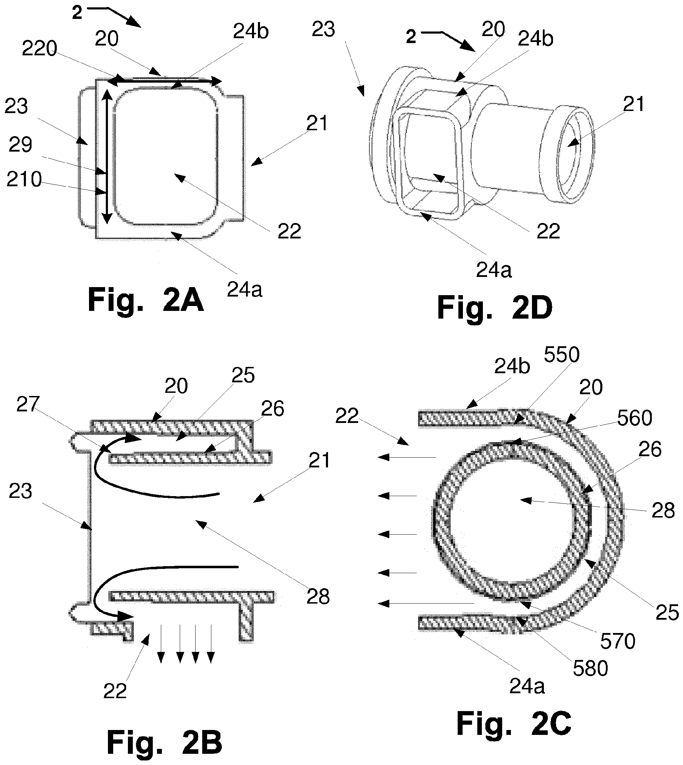

Figures 2A to 2D are illustrating an exemplary valve 2 according to the principles disclosed in connection to figure 1A to 1D. The valve 2 has a valve housing 20 with an outlet 22 and an inlet 21. The outlet has two portions 24a and 24b that go all the way to the chamber edges or close to the chamber edges.

The outlet 22 is arranged so that a portion 24a of an inner surface of outlet housing is connected with the inner surface of the valve housing 20 at a first transition portion. In some examples this means that a first transition portion, related to the portion 24a, may be arranged, in relation to the chamber 25, between a first extreme point 570 of the outer surface of the tubular channel and a first point 580 of the inner surface of the valve housing positioned at an opposite side of the chamber 25.

Depending on the direction of the valve, this may also be described as the first transition portion being arranged, in relation to the chamber 25, between a minimum point 570 of the outer surface of the tubular channel and a minimum point 580 of the inner surface of the valve housing. Additionally, the outlet 22 is arranged so that a portion 24b of an inner surface of outlet housing is connected with the inner surface of the valve housing 20 at a second transition portion. In some examples the second transition portion, related to portion 24b, is arranged between a second extreme point 560 of outer surface of said tubular channel, diametrically opposite the first extreme point 570, and a second point 550 of the inner surface of the valve housing positioned at an opposite side of the chamber 25.

Depending on the direction of the valve, this may also be described as the second transition portion being arranged, in relation to the chamber 25, between a maximum point 560 of the outer surface of the tubular channel and a maximum point 550 of the inner surface of the valve housing.

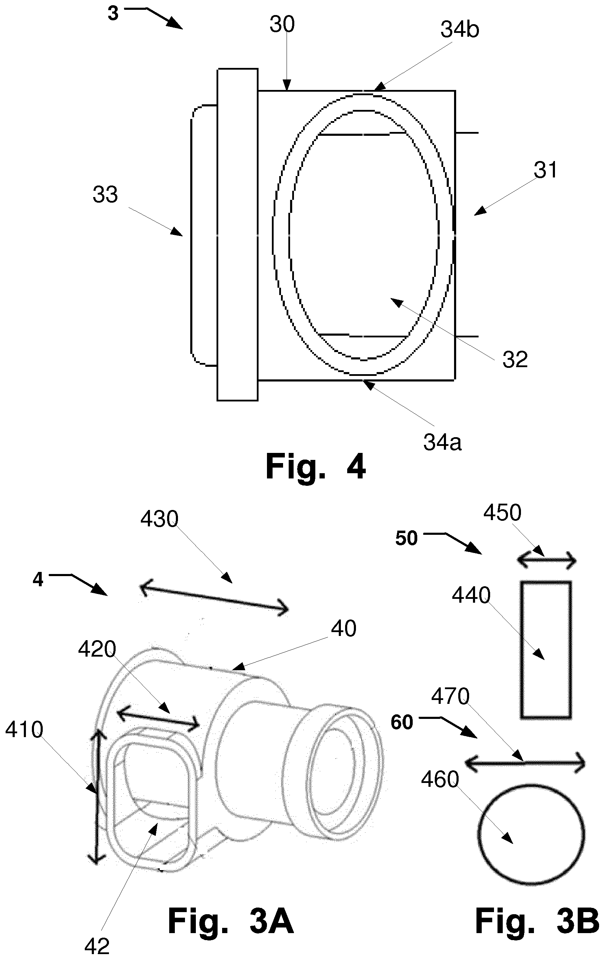

In the illustration the outlet 22 is illustrated as rectangular but other non-circular regular shapes may be possible, such as, for example, the outlet 22 being, oblong, elliptical (in a valve not according to the invention) or polygonal, as illustrated in figure 4 (showing a valve not according to the invention) .

The valve housing 20 has an inner tubular and cylindrical channel. A tubular flow channel 28 is arranged inside the valve housing 20 so that a space between an inner surface of the valve housing 20 and an outer surface 26 of the tubular flow channel 28 forms a chamber 25.

An inlet 21 is, when the valve 2 is in use, in a fluid connection via a conduit or a tube to a patient connected to a medical ventilator or anaesthesia machine.

An outlet 22 is arranged at the side of the valve housing 20, perpendicular to the inlet 21.

On the tubular flow channel 28 at an opposite side to the inlet 21 are the edges configured to be a valve seat 27. A valve body 23 rests at the valve seat 27 when the valve is closed. When the valve is open there is a gap between the valve body 23 and the valve seat 27 allowing fluid to flow from the tubular flow channel 28 into the chamber 25 and out through the outlet 22. In this example is the valve body 13 illustrated as disk-shaped, but other designs are possible. The surface of the valve body 23 in contact with the valve seat 27 should be made of a soft material.

In the examples illustrated in figures 2A to 2D, the height 210 of the outlet 22 may be about the same size as the outer size of the chamber 25, i.e. sized as the inner diameter of the valve housing 20. A large height 210 may improve the flow out from the chamber 25 as there are two portions 24a and 24b that are connected to the edges of the chamber 25, instead of one as illustrated herein in connection to the portion 14 of figures 1A to 1D. Hence the flow characteristics may be further improved.

As disclosed in relation to figures 1A to 1D having one planar side, parallel to the axial direction of the tubular flow channel 28 or valve housing, on the outlet improves the flow characteristics out from the chamber 25. Also, a wider 220 planar portion parallel to the axial direction of the tubular flow channel or valve housing, may further improve the fluid flow out of the chamber.

Additionally, in some examples of the valve 2, by arranging a planar or straight portion 29 of the outlet parallel to the valve seat 27, such as parallel to the radial direction of the flow channel or valve housing, may improve the flow characteristics out from the chamber 25, as disclosed in relation to figures 1A to 1D. The best improvement over a circular outlet is archived if the planar or straight portion 29 of the outlet 22, parallel to the valve seat 27, is the side closest to the valve seat 27. Further improvements are obtained by using two straight flow paths out from the chamber 25, such as a rectangular outlet 22 as illustrated in figures 2A to 2D. As both these planar portions 24a and 24b are connected to the edges of chamber 25, the flow resistance of the valve 2 is further reduced. The large cross-sectional flow area of such a rectangular outlet 22 lowers the pressure drop.

Additionally, when having a non-circular regular shaped outlet 22, such as a rectangular, oblong, elliptically (in a valve not according to the invention), or polygon shaped outlet, with two portions 24a and 24b that are connected to the edges of the chamber 25, a large cross sectional flow area may effectively be created, hence the homogeneity of the flow velocity may be further improved.

Additionally, due to the large cross-sectional flow area, the stability of the valve 2 may be further enhanced as the cross-sectional flow area of the chamber 25 may be further reduced.

If a round tube needs to be connected to the outlet of the valves disclosed in connection to figures 1A to 1D and figures 2A to 2D, an adapter with a rectangular shaped inlet and a round shaped outlet can be used. Alternatively if the tube or conduit is flexible it may be forced over the non-circular outlet. A round tube or a conduit connected to the non-circular outlet will have little or non-effect on the improved performance of the valve.

A check valve may be assembled with a screw outside the flow channel of the outlet. Alternatively, the check-valve may be assembled against the valve housing with an integrated molded shaft with a knob. In either case there may be nothing obstructing the flow.

Figures 3A and 3B are illustrating a schematic example of relation between areas of a round and a rectangular check-valve and the length of the valve housing. In figure 3A is a schematic disk-valve 4, according to an embodiment of the invention, illustrated. 410 is the height of the outlet 42, 420 is the width of the outlet 42 and 430 is the length of the valve housing 40.

Figure 3B is illustrating two schematic check-valves, one rectangular check-valve 50 matching the height 410 and the width 420 of the disk-valve 4 and one circular check-valve 60.

The pressure drop of a check-valve has a negative effect on the disk-valve 4. With a rectangular shape of the check-valve as in figure 3B, the effect may be less compared to a circular check-valve. The reason is that the area 440 of a rectangular check-valve 50 may be made larger than the area 460 of a circular check-valve 60 when the width 450 of the rectangular check-valve 50 is the same as the diameter 470 of a circular check-valve 60. A larger area of the check-valve will reduce the pressure-drop of the check-valve.

The disk-valve disclosed herein is more stable than a disk-valve reducing the risk of the valve to oscillate when used with a check-valve. This risk may be reduced further by using a rectangular check-valve with a larger area matching the area of the outlet of the disk-valve.

Another advantage of using a rectangular outlet and check-valve is that for a specific outlet area, such as the area 440 of the rectangular check-valve 50 is the same as the area 460 of the circular disk-valve 60. The length 430 of the valve housing 40 may be made shorter than if using a circular outlet as the width 450 may be shorter than the diameter 470.

The valve 4 may still have a reduced risk for oscillating due to the stability given by the rectangular outlet 42.

Additionally, the same applies when using, in a valve not according to the invention, an elliptically shaped outlet and check-valve.

Figure 4 is illustrating an example of a disk-valve 3, not according to the invention, having a valve housing 30, and inlet 31 and a disk shaped A valve body 33 which rests on a valve seat (not shown) when the valve is closed. The disk-valve 3 has an elliptic outlet 32 where the outlet housing goes from one edge 34a of the chamber to another edge 34b of the chamber.

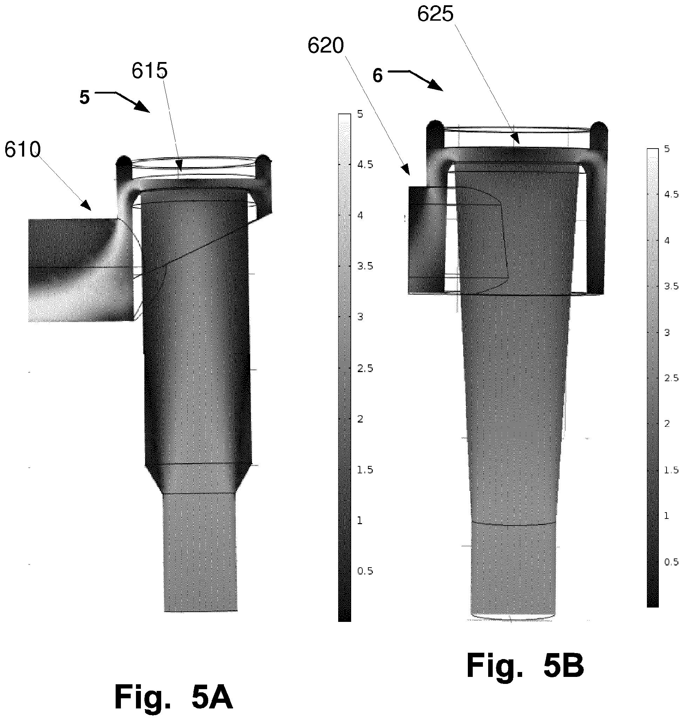

Figure 5A and 5B illustrate two valves 5, 6. Figure 5A illustrates a valve 5, not according to the invention, with a circular outlet 610 and figure 5B illustrates a valve 6, according to an embodiment of the invention, with a rectangular outlet 620. For these simulations the velocity was 30 l/min and the opening valve gap 2.5 mm. On the scale 0 means low turbulence and 5 means higher turbulence. As seen, the circular outlet model 5 has a larger velocity concentrated to the lower part of the outlet tube 610 while the rectangular outlet model 6 has a more homogeneous outlet flow through the outlet 620. The flow at the valve seat 615 is concentrated at the closest way to the outlet 610 for the circular outlet model 5. But for the rectangular outlet model 6 the flow is spread out all around the valve seat 625. The more homogenous velocity experienced in figure 5B is due to the rectangular shape combined with the large outlet that goes all the way out to the chamber edges.

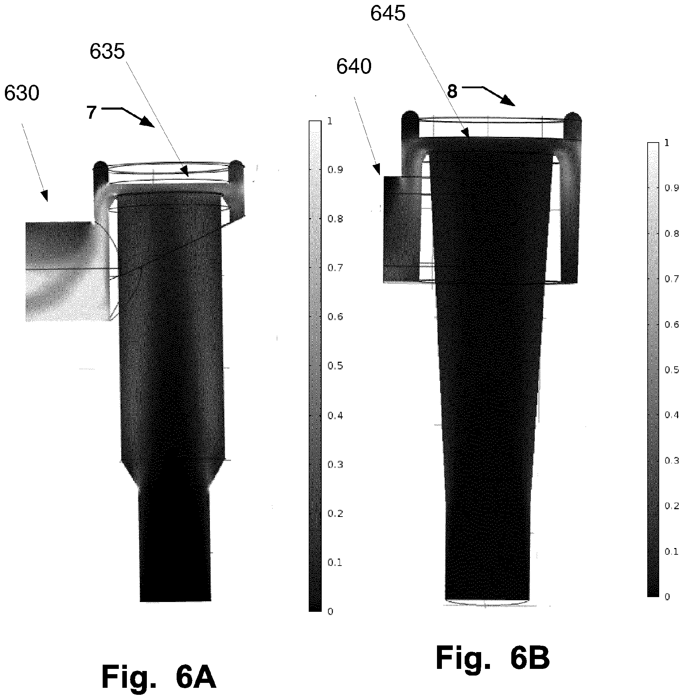

Figure 6A and 6B illustrate two valves 7, 8. Figure 6A illustrates a valve 7, not according to the invention, with a circular outlet 630 and figure 6B illustrates a valve 8, according to an embodiment of the invention, with a rectangular outlet 640. For these simulations the velocity was 30 l/min and the opening valve gap 2.5 mm. On the scale 0 means low turbulence and 1 means higher turbulence.

Figure 6A and 6B illustrates the turbulent kinetic energy. The rectangular shaped model 8 has a much lower turbulence level at the outlet 640 and at the valve seat 645 compared to the turbulence level at the circular outlet 630 and the related valve set 635. A low turbulence at the outlet can be good if you for example want to attach a flow sensor after the valve outlet. A homogenous turbulence around the valve seat is good for the stability of the valve.

| | Rectangular outlet | Circular |
|---|---|---|
| Lit/min | Δp (mBar) | Δp (mBar) |
| 30 | 0.14 | 0.3 |
| 60 | 0.53 | |
| 150 | 3.3 | 6.5 |
| 180 | 4.7 | |

The table above show simulated pressure drops of a rectangular shaped and a circular shaped outlet at different flows. Tests have showed that the simulated pressure drop is very close to real measured values.

Herein disclosed is also a method of reducing flow resistance of a disk-valve for a respiratory device. The method comprising providing a non-circular regular shaped outlet to a disk-valve, such as a disk-valve disclosed herein above. The outlet is arranged perpendicular to an inlet. This method related to manufacturing a disk-valve either by fabricating a disk-valve with a non-circular regular shaped outlet, or by modifying an outlet of a pre-existing disk-valve to be non-circular regular shaped. The disk-valve is a disk-valve as herein disclosed above.

## Claims

1. A valve (1, 2, 4, 6, 8) for a respiratory device, comprising:
a valve housing (10, 20) having an inlet (11, 21) and an outlet (12, 22, 620, 640);
a tubular flow channel (18, 28) arranged in said valve housing (10, 20), one end of said tubular flow channel (10, 20) being said inlet (11, 21) and an edge at the other end of said tubular flow channel (10, 20) being configured as a valve seat (17, 27, 625, 645);
a chamber (15, 25) arranged between a cylindrical inner surface of said valve housing (10, 20) and an outer surface (16, 26) of said tubular flow channel (18, 28); and
a valve body (13, 23) configured such that, in use, said valve body abuts said valve seat (17, 27, 625, 645) when said valve is closed, and when said valve is open, a gap is formed between said valve seat (17, 27, 625, 645) and said valve body (13, 23) allowing a fluid communication from said tubular flow channel (18, 28) into said chamber (15, 25) and out through said outlet (12, 22, 640),
wherein:
said outlet (12, 22, 620, 640) has an outlet housing; and
said outlet (12, 22, 620, 640) is arranged perpendicular to said inlet (11, 21),
**characterised in that:**
said outlet (12, 22, 620, 640) has a rectangular, an oblong, a square, or a polygonal shape; and
at least one portion of said outlet housing is:
planar;
arranged parallel to an axial direction of said tubular flow channel (18, 28) and said valve housing (10, 20); and connected to the inner cylindrical surface of the valve housing so that the inner surface of the at least one planar portion of the outlet housing is arranged tangential to the cylindrical inner surface of the valve housing, such that a fluid can flow out from the chamber in a tangent direction along the inner surface of the at least one portion of the outlet housing.

2. The valve according to claim 1, wherein said outlet (12, 22, 620, 640) is arranged so that said inner surface of said at least one portion of said outlet housing is connected with said inner surface of said valve housing (10, 20) at a first transition portion, and wherein said first transition portion is arranged, in relation to said chamber (15, 25), between a first extreme point (520, 530) of said outer surface of said tubular flow channel (18, 28) and a first point (510, 540) of said inner surface of said valve housing (10, 20) positioned at an opposite side of said chamber (15, 25).

3. The valve according to claim 2, wherein said first transition portion is located inside said chamber (15, 25) so that said inner surface of said first portion of said outlet housing extends out from said chamber (15, 25).

4. The valve according to any of claims 1 to 3, wherein said inner surface of said at least one portion of said outlet housing allows a flow of a fluid out from said chamber (15, 25) in a tangent direction along said inner surface of said at least one portion of said outlet housing.

5. The valve according to any of claims 1 to 4, wherein a second portion of the inner surface of said outlet housing is connected with said inner surface of said valve housing at a second transition portion, and wherein said second transition portion is arranged between a second extreme point (560) of said outer surface of said tubular flow channel (18, 28), diametrically opposite said first extreme point (570), and a second point (550) of said inner surface of said valve housing (10, 20) positioned at an opposite side of said chamber (15, 25).

6. The valve according to claim 5, wherein a height of said outlet (12, 22, 620, 640) is about the same as an inner diameter of said valve housing (10, 20).

7. The valve according to any of claims 1 to 6, wherein one further portion of said outlet (12, 22, 620, 640) is planar or straight, and arranged parallel to a radial direction of said tubular flow channel (18, 28), or said valve housing (10, 20).

8. The valve according to any of claims 1 to 7, wherein said valve is an expiration valve or an inspiration valve.

9. The valve according to any of claims 1 to 8, wherein said outlet (12, 22, 620, 640) has a check valve connected to it.

10. A kit comprising:
a valve according to any of claims 1 to 9; and
an adaptor for connecting a tube to an outlet (12, 22, 620, 640) of said valve, said adaptor having a rectangular, an oblong, a square, or a polygonal shape.

11. A method of reducing flow resistance of a disk-valve for a respiratory device, the method comprising providing an outlet to said disk-valve, said outlet having a rectangular, an oblong, a square, or a polygonal shape, wherein:
said disk-valve comprises:
a valve housing (10, 20) having an inlet (11, 21) and an outlet (12, 22, 620, 640) ;
a tubular flow channel (18, 28) arranged in said valve housing (10, 20), one end of said tubular flow channel (10, 20) being said inlet (11, 21) and an edge at the other end of said tubular flow channel (10, 20) being configured as a valve seat (17, 27, 625, 645);
a chamber (15, 25) arranged between an inner surface of said valve housing (10, 20) and an outer surface (16, 26) of said tubular flow channel (18, 28); and
a valve body (13, 23) configured such that, in use, said valve body abuts said valve seat (17, 27, 625, 645) when said valve is closed, and when said valve is open, a gap is formed between said valve seat (17, 27, 625, 645) and said valve body (13, 33) allowing a fluid communication from said tubular flow channel (18, 28) into said chamber (15, 25) and out through said outlet (12, 22, 620, 640); and
wherein the outlet is provided such that:
said outlet (12, 22, 620, 640) has an outlet housing;
said outlet (12, 22, 620, 640) is arranged perpendicular to said inlet (11, 21); and
at least one portion of said outlet housing is:
planar;
arranged parallel to an axial direction of said tubular flow channel (18, 28) and said valve housing (10, 20); and
connected to the inner cylindrical surface of the valve housing so that the inner surface of the at least one planar portion of the outlet housing is arranged tangential to the cylindrical inner surface of the valve housing, such that a fluid can flow out from the chamber in a tangent direction along the inner surface of the at least one portion of the outlet housing.

12. The method according to claim 11, wherein said outlet is provided so that said inner surface of said at least one portion of said outlet housing is connected with said inner surface of said valve housing at a first transition portion, and wherein said first transition portion is arranged, in relation to said chamber, between an extreme point of an outer surface of said tubular channel and a point of said inner surface of said valve housing positioned at an opposite side of said chamber.

## Patentansprüche

1. Ein Ventil (1, 2, 4, 6, 8) für ein Beatmungsgerät mit:
einem Ventilgehäuse (10, 20) mit einem Einlass (11, 21) und einem Auslass (12, 22, 620, 640),
einem schlauchförmigen Strömungskanal (18, 28), der im vorerwähnten Ventilgehäuse (10, 20) angeordnet ist,
wobei ein Ende des vorerwähnten schlauchförmigen Strömungskanals (10, 20) der vorerwähnte Einlass (11, 21) ist und eine Kante am anderen Ende des vorerwähnten schlauchförmigen Strömungskanals (10, 20) als Ventilsitz (17, 27, 625, 645) ausgebildet ist,
einer Kammer (15, 25), die zwischen einer zylindrischen Oberfläche des vorerwähnten Ventilgehäuses (10, 20) und einer Außenfläche (16, 26) des vorerwähnten schlauchförmigen Strömungskanals (18, 28) ausgebildet ist, und
einem Ventilkörper (13, 23), der so ausgelegt ist, dass der vorerwähnte Ventilkörper im Betrieb bei geschlossenem Ventil an den vorerwähnten Ventilsitz (17, 27, 625, 645) angrenzt und bei offenem Ventil ein Spalt zwischen dem vorerwähnten Ventilsitz (17, 27, 625, 645) und dem vorerwähnten Ventilkörper (13, 23) gebildet wird, der eine Fließverbindung vom vorerwähnten schlauchförmigen Strömungskanal (18, 28) in die vorerwähnte Kammer (15, 25) und über den vorerwähnten Auslass (12, 22, 620, 640) ermöglicht, wobei:
der vorerwähnte Auslass (12, 22, 620, 640) ein Auslassgehäuse aufweist und
der vorerwähnte Auslass (12, 22, 620, 640) senkrecht zum vorerwähnten Einlass (11, 21) angeordnet ist,
**dadurch gekennzeichnet, dass** der vorerwähnte Auslass (12, 22, 620, 640) eine rechteckige, längliche, quadratische oder mehreckige Form besitzt und
mindestens ein Bereich des vorerwähnten Auslassgehäuses:
eben ist,
parallel zu einer axialen Richtung des vorerwähnten schlauchförmigen Strömungskanals (18, 28) und des vorerwähnten Ventilgehäuses (10, 20) angeordnet ist und
mit der zylindrischen Innenfläche des Ventilgehäuses verbunden ist, so dass die Innenfläche des mindestens einen ebenen Bereichs des Auslassgehäuses tangential zur zylindrischen Innenfläche des Ventilgehäuses angeordnet ist, so dass eine Flüssigkeit in tangentialer Richtung entlang der Innenfläche des mindestens einen Bereichs des Auslassgehäuses aus der Kammer fließen kann.

2. Das Ventil gemäß Anspruch 1, wobei der vorerwähnte Auslass (12, 22, 620, 640) so angeordnet ist, dass die vorerwähnte Innenfläche des mindestens einen Bereichs des vorerwähnten Auslassgehäuses mit der vorerwähnten Innenfläche des vorerwähnten Ventilgehäuses (10, 20) in einem ersten Übergangsbereich in Verbindung steht, wobei der vorerwähnte erste Übergangsbereich in Bezug auf die vorerwähnte Kammer (15, 25) zwischen einem ersten Endpunkt (520, 530) der vorerwähnten Außenfläche des vorerwähnten schlauchförmigen Strömungskanals (18, 28) angeordnet und ein erster Punkt (510, 540) der vorerwähnten Innenfläche des vorerwähnten Ventilgehäuses (10, 20) am entgegengesetzten Ende der vorerwähnten Kammer (15, 25) positioniert ist.

3. Das Ventil gemäß Anspruch 2, wobei der vorerwähnte erste Übergangsbereich sich innerhalb der vorerwähnten Kammer (15, 25) befindet, so, dass die vorerwähnte Innenfläche des vorerwähnten ersten Bereichs des vorerwähnten Auslassgehäuses über die vorerwähnte Kammer (15, 25) hinausragt.

4. Das Ventil gemäß einem der Ansprüche 1 bis 3, wobei die vorerwähnte Innenfläche des mindestens einen Bereichs des vorerwähnten Auslasses einen Flüssigkeitsstrom aus der vorerwähnten Kammer (15, 25) in tangentialer Richtung entlang der vorerwähnten Innenfläche des mindestens einen Bereichs des vorerwähnten Auslassgehäuses gestattet.

5. Das Ventil gemäß einem der Ansprüche 1 bis 4, wobei ein zweiter Bereich der Innenfläche des vorerwähnten Auslassgehäuses in einem zweiten Übergangsbereich mit der vorerwähnten Innenfläche des vorerwähnten Ventilgehäuses verbunden ist und
wobei der vorerwähnte zweite Übergangsbereich zwischen einem zweiten Endpunkt (560) der vorerwähnten Außenfläche des vorerwähnten schlauchförmigen Strömungskanals (18, 28) diametral zum vorerwähnten ersten Endpunkt (570) liegt und ein zweiter Punkt (550) der vorerwähnten Innenfläche des vorerwähnten Ventilgehäuses (10, 20) an einer gegenüberliegenden Seite der vorerwähnten Kammer (15, 25) positioniert ist.

6. Das Ventil gemäß Anspruch 5, wobei die Höhe des vorerwähnten Auslasses (12, 22, 620, 640) in etwa dem Innendurchmesser des vorerwähnten Ventilgehäuses (10, 20) entspricht.

7. Das Ventil gemäß einem der Ansprüche 1 bis 6, wobei ein weiterer Bereich des vorerwähnten Auslasses (12, 22, 620, 640) eben oder glatt ausgeformt und parallel zur radialen Richtung des vorerwähnten schlauchförmigen Strömungskanals (18, 28) oder dem vorerwähnten Ventilgehäuse (10, 20) angeordnet ist.

8. Das Ventil gemäß einem der Ansprüche 1 bis 7, wobei es sich bei dem vorerwähnten Ventil um ein Ausatemventil oder ein Einatemventil handelt.

9. Das Ventil gemäß einem der Ansprüche 1 bis 8, wobei ein Rückschlagventil an den vorerwähnten Auslass (12, 22.620, 640) angeschlossen ist.

10. Ein Set mit:
einem Ventil gemäß einem der Ansprüche 1 bis 9 und einem Adapter für den Anschluss eines Schlauchs an den Auslass (12, 22, 320, 640) des vorerwähnten Ventils, wobei der vorerwähnte Adapter eine rechteckige, längliche, quadratische oder mehreckige Form besitzt.

11. Ein Verfahren zur Reduzierung des Strömungswiderstands eines Tellerventils für ein Beatmungsgerät, wobei das Verfahren einen Auslass zum vorerwähnten Tellerventil aufweist und der vorerwähnte Auslass eine rechteckige, längliche, quadratische oder mehreckige Form besitzt, wobei das vorerwähnte Tellerventil folgendes aufweist:
ein Ventilgehäuse (10, 20) mit einem Einlass (11, 21) und einem Auslass (12, 22, 620, 640),
einen innerhalb des vorerwähnten Ventilgehäuses (10, 20) angeordneten schlauchförmigen Strömungskanal, wobei ein Ende des vorerwähnten schlauchförmigen Strömungskanals (10, 20) der vorerwähnte Einlass (11, 21) ist und eine Kante am anderen Ende des vorerwähnten schlauchförmigen Strömungskanals (10, 20) als Ventilsitz (17, 27, 625, 645) ausgebildet ist,
eine Kammer (15, 25), die zwischen einer Oberfläche des vorerwähnten Ventilgehäuses (10, 20) und einer Außenfläche (16, 26) des vorerwähnten schlauchförmigen Strömungskanals (18, 28) ausgebildet ist, und
ein Ventilkörper (13, 23), der so ausgelegt ist, dass der vorerwähnte Ventilkörper im Betrieb bei geschlossenem Ventil an den vorerwähnten Ventilsitz (17, 27, 625, 645) angrenzt und bei offenem Ventil ein Spalt zwischen dem vorerwähnten Ventilsitz (17, 27, 625, 645) und dem vorerwähnten Ventilkörper (13, 33) gebildet wird, der eine Fließverbindung vom vorerwähnten schlauchförmigen Strömungskanal (18, 28) in die vorerwähnte Kammer (15, 25) und über den vorerwähnten Auslass (12, 22, 620, 640) ermöglicht, und
wobei der Auslass so ausgelegt ist, dass:
der vorerwähnte Auslass (12, 22, 620, 640) ein Auslassgehäuse aufweist,
der vorerwähnte Auslass (12, 22, 620, 640) senkrecht zum vorerwähnten Einlass (11, 21) angeordnet ist
und mindestens ein Bereich des vorerwähnten Auslassgehäuses
eben ist,
parallel zu einer axialen Richtung des vorerwähnten schlauchförmigen Strömungskanals (18, 28) und des vorerwähnten Ventilgehäuses (10, 20) angeordnet ist und
mit der inneren zylindrischen Oberfläche des Ventilgehäuses verbunden ist, so dass die Innenfläche des mindestens einen ebenen Bereichs des Auslassgehäuses tangential zur zylindrischen Innenfläche des Ventilgehäuses angeordnet ist, so dass eine Flüssigkeit in tangentialer Richtung entlang der Innenfläche des mindestens einen Bereichs des Auslassgehäuses aus der Kammer fließen kann.

12. Das Verfahren gemäß Anspruch 11, wobei der vorerwähnte Auslass so angeordnet ist, dass die vorerwähnte Innenfläche des mindestens einen Bereichs des vorerwähnten Auslassgehäuses mit der vorerwähnten Innenfläche des vorerwähnten Ventilgehäuses in einem ersten Übergangsbereich in Verbindung steht und wobei der vorerwähnte erste Übergangsbereich in Bezug auf die vorerwähnte Kammer zwischen einem Endpunkt der vorerwähnten Außenfläche des vorerwähnten schlauchförmigen Strömungskanals angeordnet und ein Punkt der vorerwähnten Innenfläche des vorerwähnten Ventilgehäuses am entgegengesetzten Ende der vorerwähnten Kammer positioniert ist.

## Revendications

1. Valve (1, 2, 4, 6, 8) destinée à un dispositif d'assistance respiratoire, comprenant :
un logement de valve (10, 20) présentant une entrée (11, 21) et une sortie (12, 22, 620, 640);
un canal d'écoulement tubulaire (18, 28) agencé dans ledit logement de valve (10, 20), une première extrémité dudit canal d'écoulement tubulaire (10, 20) étant ladite entrée (11, 21) et un bord au niveau de l'autre extrémité dudit canal d'écoulement tubulaire (10, 20) étant configuré comme un siège de valve (17, 27, 625, 645) ;
une chambre (15, 25) agencée entre une surface intérieure cylindrique dudit logement de valve (10, 20) et une surface extérieure (16, 26) dudit canal d'écoulement tubulaire (18, 28) ; et
un corps de valve (13, 23) configuré de telle sorte que, en utilisation, ledit corps de valve est en butée contre ledit siège de valve (17, 27, 625, 645) lorsque ladite valve est fermée, et lorsque ladite valve est ouverte, un intervalle est formé entre ledit siège de valve (17, 27, 625, 645) et ledit corps de valve (13, 23) permettant une communication de fluide depuis ledit canal d'écoulement tubulaire (18, 28) jusque dans ladite chambre (15, 25) et hors de celle-ci par le biais de ladite sortie (12, 22, 620, 640), où :
ladite sortie (12, 22, 620, 640) présente un logement de sortie ; et
ladite sortie (12, 22, 620, 640) est agencée perpendiculairement par rapport à ladite entrée (11, 21),
**caractérisée en ce que :**
ladite sortie (12, 22, 620, 640) présente une forme rectangulaire, oblongue, carrée ou polygonale ; et
au moins une partie dudit logement de sortie est :
plane ;
agencée parallèlement à une direction axiale dudit canal d'écoulement tubulaire (18, 28) et dudit logement de valve (10, 20) ; et
reliée à la surface cylindrique intérieure dudit logement de valve de sorte que la surface intérieure de la au moins une partie plane du logement de sortie est agencée de manière à être tangentielle à la surface intérieure cylindrique du logement de valve, de telle sorte qu'un fluide peut s'écouler hors de la chambre dans une direction tangentielle le long de la surface intérieure de la au moins une partie du logement de sortie.

2. Valve selon la revendication 1, dans laquelle ladite sortie (12, 22, 620, 640) est agencée de sorte que ladite surface intérieure de ladite au moins une partie dudit logement de sortie est reliée avec ladite surface intérieure dudit logement de valve (10, 20) au niveau d'une première partie de transition, et où ladite première partie de transition est agencée, par rapport à ladite chambre (15, 25), entre un premier point extrême (520, 530) de ladite surface extérieure dudit canal d'écoulement tubulaire (18, 28) et un premier point (510, 540) de ladite surface intérieure dudit logement de valve (10, 20) positionné au niveau d'un côté opposé de ladite chambre (15, 25).

3. Valve selon la revendication 2, dans laquelle ladite première partie de transition est située à l'intérieur de ladite chambre (15, 25) de sorte que ladite surface intérieure de ladite première partie dudit logement de sortie s'étend hors de ladite chambre (15, 25).

4. Valve selon l'une quelconque des revendications 1 à 3, dans laquelle ladite surface intérieure de ladite au moins une partie dudit logement de sortie permet un écoulement d'un fluide hors de ladite chambre (15, 25) dans une direction tangentielle le long de ladite surface intérieure de ladite au moins une partie dudit logement de sortie.

5. Valve selon l'une quelconque des revendications 1 à 4, dans laquelle une deuxième partie de la surface intérieure dudit logement de sortie est reliée à ladite surface intérieure dudit logement de valve au niveau d'une deuxième partie de transition, et où ladite deuxième partie de transition est agencée entre un deuxième point extrême (560) de ladite surface extérieure dudit canal d'écoulement tubulaire (18, 28), diamétralement opposé audit premier point extrême (570), et un deuxième point (550) de ladite surface intérieure dudit logement de valve (10, 20) positionné au niveau d'un côté opposé de ladite chambre (15, 25).

6. Valve selon la revendication 5, dans laquelle une hauteur de ladite sortie (12, 22, 620, 640) est approximativement identique à un diamètre intérieur dudit logement de valve (10, 20).

7. Valve selon l'une quelconque des revendications 1 à 6, dans laquelle une autre partie de ladite sortie (12, 22, 620, 640) est plane ou droite, et agencée parallèlement à une direction radiale dudit canal d'écoulement tubulaire (18, 28), ou dudit logement de valve (10, 20).

8. Valve selon l'une quelconque des revendications 1 à 7, où ladite valve est une valve d'expiration ou une valve d'inspiration.

9. Valve selon l'une quelconque des revendications 1 à 8, dans laquelle ladite sortie (12, 22, 620, 640) présente un clapet anti-retour relié à celle-ci.

10. Ensemble comprenant :
une valve selon l'une quelconque des revendications 1 à 9 ; et
un adaptateur destiné à relier un tube à une sortie (12, 22, 620, 640) de ladite valve, ledit adaptateur présentant une forme rectangulaire, oblongue, carrée ou polygonale.

11. Procédé de réduction de résistance à l'écoulement d'une valve à disque destinée à un dispositif d'assistance respiratoire, le procédé comprenant le fait de fournir une sortie à ladite valve à disque, ladite sortie présentant une forme rectangulaire, oblongue, carrée ou polygonale,
où :
ladite valve à disque comprend :
un logement de valve (10, 20) présentant une entrée (11, 21) et une sortie (12, 22, 620, 640);
un canal d'écoulement tubulaire (18, 28) agencé dans ledit logement de valve (10, 20), une première extrémité dudit canal d'écoulement tubulaire (10, 20) étant ladite entrée (11, 21) et un bord au niveau de l'autre extrémité dudit canal d'écoulement tubulaire (10, 20) étant configuré comme un siège de valve (17, 27, 625, 645) ;
une chambre (15, 25) agencée entre une surface intérieure dudit logement de valve (10, 20) et une surface extérieure (16, 26) dudit canal d'écoulement tubulaire (18, 28) ; et
un corps de valve (13, 23) configuré de telle sorte que, en utilisation, ledit corps de valve est en butée contre ledit siège de valve (17, 27, 625, 645) lorsque ladite valve est fermée, et lorsque ladite valve est ouverte, un intervalle est formé entre ledit siège de valve (17, 27, 625, 645) et ledit corps de valve (13, 33) permettant une communication de fluide depuis ledit canal d'écoulement tubulaire (18, 28) jusque dans ladite chambre (15, 25) et hors de celle-ci par le biais de ladite sortie (12, 22, 620, 640) ; et
où la sortie est prévue de telle sorte que :
ladite sortie (12, 22, 620, 640) présente un logement de sortie ;
ladite sortie (12, 22, 620, 640) est agencée perpendiculairement par rapport à ladite entrée (11, 21) ; et
au moins une partie dudit logement de sortie est :
plane ;
agencée parallèlement à une direction axiale dudit canal d'écoulement tubulaire (18, 28) et dudit logement de valve (10, 20) ; et
reliée à la surface cylindrique intérieure dudit logement de valve de sorte que la surface intérieure de la au moins une partie plane du logement de sortie est agencée de manière à être tangentielle à la surface intérieure cylindrique du logement de valve, de telle sorte qu'un fluide peut s'écouler hors de la chambre dans une direction tangentielle le long de la surface intérieure de la au moins une partie du logement de sortie.

12. Procédé selon la revendication 11, dans lequel ladite sortie est prévue de telle sorte que ladite surface intérieure de ladite au moins une partie dudit logement de sortie est reliée à ladite surface intérieure dudit logement de valve au niveau d'une première partie de transition, et où ladite première partie de transition est agencée, par rapport à ladite chambre, entre un point extrême d'une surface extérieure dudit canal tubulaire et un point de ladite surface intérieure dudit logement de valve positionné au niveau d'un côté opposé de ladite chambre.
